# EUROPÄISCHE PATENTSCHRIFT

(11) **EP 1 933 693 B1**
(45) Veröffentlichungstag und Bekanntmachung des Hinweises auf die Patenterteilung: **28.05.2014**
(21) Anmeldenummer: 06791821.9
(22) Anmeldetag: 05.09.2006
(51) Int. Cl.: A61B 3/103, A61B 3/13, A61B 19/00, G02B 21/00

(54) **OPHTHALMO-OPERATIONSMIKROSKOP MIT MESSEINRICHTUNG**
OPHTHALMIC OPERATING MICROSCOPE COMPRISING A MEASURING UNIT
MICROSCOPE D'OPERATION OPHTALMIQUE COMPORTANT UN DISPOSITIF DE MESURE

(30) Priorität: 07.09.2005 DE 102005042436
(43) Veröffentlichungstag der Anmeldung: 25.06.2008
(73) Patentinhaber: Carl Zeiss Meditec AG, 07745 Jena (DE)
(72) Erfinder: GAIDA, Gerhard, 73430 Aalen (DE)
(74) Vertreter: Carl Zeiss AG - Patentabteilung
(86) Internationale Anmeldenummer: PCT/EP2006/008616
(87) Internationale Veröffentlichungsnummer: WO 2007/028564

(56) Entgegenhaltungen:
- EP-A1- 0 247 260
- EP-A2- 1 338 238
- WO-A-03/030763
- WO-A-2004/003468
- US-A1- 2004 246 440

## Beschreibung

Die Erfindung betrifft ein Operationsmikroskop für die Ophthalmologie mit einer Messeinrichtung zum Bestimmen von wenigstens einer optischen Kenngröße eines Patientenauges.

Vorliegend wird unter einer optischen Kenngröße eines Patientenauges die geometrische Länge zwischen optischen wirksamen Flächen des Auges verstanden, an denen ein Sprung oder ein nicht verschwindender Gradient des Brechungsindex auftritt, oder an denen eine Absorption von Licht erfolgt. Die lokalen Radien der Flächen, d.h. deren Gauß'sche Krümmungen können allerdings ebenfalls als "optische Kenngrößen" eines Patientenauges in diesem Sinne aufgefasst werden. Eine optische Kenngröße eines Patientenauges ist im übrigen auch der Brechungsindex oder die Brechkraft eines optisch wirksamen Bestandteils darin.

Ein Operationsmikroskop der eingangs genannten Art ist aus der EP 0 697 611 B1 bekannt. Dort ist ein Operationsmikroskop mit einem Kohärenzinterferometer in Form einer OCT-Messeinrichtung beschrieben. Diese OCT-Messeinrichtung stellt einen OCT-Abtaststrahl bereit, der in transversaler Richtung über die Kornea eines Patientenauges gescannt werden kann, um so deren Dicke zu bestimmen und den genauen Verlauf von Vorder- und Hinterfläche der Kornea zu vermessen.

Aus der DE 102 02 509 A1 ist ein Ophthalmo-Operationsmikroskop mit integriertem Refraktometer bekannt. Mittels dieses Refraktometers kann im laufenden Operationsbetrieb die optische Eigenschaft einer bei einer Kateraktoperation eingesetzten künstlichen Augenlinse überwacht werden, so dass es möglich ist, diese bei Bedarf zu korrigieren.

Die US 5,828,439 offenbart ein Refraktometer, mit dem die Brechkraft des menschlichen Auges während eines chirurgischen Eingriffs bestimmt werden kann. In dieses Refraktometer ist eine Messeinrichtung zur Ermittlung einer nichtoptischen Kenngröße des Auges in Form des Augeninnendrucks integriert. Dieser Augeninnendruck wird herangezogen, um einen gemessenen Brechkraftwert zu korrigieren.

Die DE 43 10 561 A1 offenbart eine Vorrichtung zur Bestimmung von Refraktion und Sehschärfe während eines chirurgischen Eingriffs am menschlichen Auge. Diese Vorrichtung gestattet, auf die Netzhaut des Patientenauges eine mittels Lichtquelle beleuchtete Testkarte abzubilden, welche sich zur Messung einer Abbildungsschärfe des Auges eignet. Erkennt ein Arzt mit der Vorrichtung eine scharfe Testkartenabbildung auf der Patientenauge-Netzhaut, so kann aus den Geräteparametern der Vorrichtung ein Refraktionswert für das untersuchte Auge absolut bestimmt werden. Die Vorrichtung ist mit einem Operationsmikroskop kombiniert und ermöglicht so wiederum einem Arzt bei Betrachtung des Patientenauges eine Refraktionsbestimmung im laufenden Operationsbetrieb.

In der EP 0 247 260 B1 ist eine Vorrichtung zur Analyse und Korrektur der Brechkraft eines menschlichen Auges beschrieben. Die Vorrichtung umfasst einen Datenspeicher, in dem Daten eines idealisierten Patientenauges für unterschiedliche axiale Augenlängen, Patientenalter, Geschlecht aber auch Werte für den Augeninnendruck abgelegt sind. In der Vorrichtung sind Mittel zum Bestimmen der aktuellen Hornhauttopographie vorgesehen. Die Vorrichtung umfasst weiter eine Lasereinheit, mit der Hornhaut des menschlichen Auges definiert abgetragen werden kann. Der Abtrag wird dabei so eingestellt, dass sich eine gewünschte Topographie eines idealisierten Patientenauges ergibt. Die Entwicklung der Kataraktchirurgie zu immer schonenderen Operationstechniken einhergehend mit der refraktiven Chirurgie der Hornhaut hat dazu geführt, dass die Ansprüche an die optischen Eigenschaften eines Patientenauges nach der Kataraktoperation gestiegen sind. Es wird gefordert, dass die volle Sehkraft ohne korrigierende Brille erreicht wird.

Weiter wird in der ophthalmologischen Chirurgie versucht, Sehkraftschwächen des menschlichen Auges zu korrigieren, wenn dessen Pupillenweite größer als 2mm beträgt. Diese Sehkraftschwächen haben ihre Ursache in der von Natur aus ungenügenden geometrisch-optischen Korrektur des Auges. Hier wird versucht, durch Eingriffe in die optischen Eigenschaften eines Patientenauges Patienten mit durchschnittlicher oder unterdurchschnittlicher Sehkraft zu einer überdurchschnittlichen Sehschärfe, d.h. Visus > 1, zu verhelfen, wie sie nur in Ausnahmefällen bei Menschen anzutreffen ist.

Schließlich gibt es in der ophthalmologischen Chirurgie Bestrebungen, das Nachlassen der Akkomodationsfähigkeit des menschlichen Auges im Alter, die sogenannte Presbyopie, umzukehren. Da Effektoren der Akkomodation die sogenannten Ziliarmuskeln sind, besteht ein Weg, die Akkomodationsfähigkeit des Auges im Alter zu verbessern, darin, die alternde natürliche Linse des Auges gegen eine künstliche auszutauschen.

Vor diesem Hintergrund ist es für die ophthalmologische Chirurgie wünschenswert und notwendig, dass der refraktive Zustand eines Patientenauges nach einer Operation, insbesondere dessen Zustand im Augeninnern und der Zustand der Augenlinse, möglichst genau vorherzusagen. Für eine solche Vorhersage sind präzise optische Messmethoden bekannt und können beispielsweise mit dem IOL Master der Carl Zeiss Meditec AG im Rahmen eine präoperativen Charakterisierung des Auges durchgeführt werden.

Beim gegenwärtigen Stand der Kataraktchirurgie wird jedoch nur eine aufgrund von präoperativen Daten ausgesuchte Intraokularlinse eingesetzt. Dann wird die Wundheilung des Auges abgewartet, während der sich optische Kenngrößen, wie Abstände der brechenden Bestandteile des Auges, häufig verändern. Erst nach Abschluss der Wundheilung stabilisieren sich die optischen Eigenschaften des Auges.

Es ist vor diesem Hintergrund wünschenswert, die optischen Eigenschaften des Auges auch während einer Operation zu kontrollieren, um so schnell eine unerwünschte Abweichung von einem vorgeplanten Zustand erkennen zu können. Dann besteht nämlich die Möglichkeit, die Operation abzuwandeln. Darüber hinaus gibt es mittlerweile Operationstechniken, beispielsweise injizierbare intraokulare Linsen, die es notwendig machen, optische Eigenschaften des Auges während einer Operation abzugleichen. Bislang ist es im Stand der Technik lediglich bekannt, eine einzige optische Größe des Auges, beispielsweise dessen Brechkraft, intraoperativ zu messen. Aufgrund der Grundlage dieser einzigen optischen Kenngröße lässt sich jedoch der optische Zustand des Auges nicht zuverlässig bestimmen. Es zeigt sich nämlich, dass sich im Verlauf eines Heilungsprozesses nach einem chirurgischen Eingriff am Auge nicht nur eine einzige optisch wirksame Größe ändert, sondern es findet eine Modifikation mehrere optischer Kenngrößen des Auges statt, deren optische Wirkung sich möglicherweise teilweise verstärkt aber auch gegenseitig abschwächt.

Bei dem menschlichen Auge handelt es sich um ein optisches System, das in der Regel weit davon entfernt ist, perfekt zu sein. In einem perfekten Auge, das auch als emmetropisches Auge bezeichnet wird, konvergieren Lichtstrahlen, die von einem Punkt in einem Objektbereich herrühren, im Augeninnern in einem Punkt, der auf der Retina des Auges liegt. In der Wirklichkeit kommt dieser Zustand jedoch nie vor. Zum einen liegt dies daran, dass ein optisches System, welches eine begrenzte Apertur aufweist, aufgrund der Beugung des Lichts einen mathematischen Punkt, dessen Durchmesser streng genommen Null beträgt, auf einen Punkt abbildet, dessen Durchmesser größer als Null ist. Zum anderen liegt dies daran, dass die optischen Komponenten des Auges, nämlich Cornea und Linse, weit davon entfernt sind, perfekt zu sein. Wenn die Krümmung der Cornea zu groß oder das Auge zu lang ist, so liegt das Bild eines Objektes in einer Ebene, die sich vor der Retina befindet. Dies führt dazu, dass das Objekt mit dem Auge verschwommen wahrgenommen wird. Der entsprechende Sehfehler wird als "Myopie" bezeichnet. Umgekehrt, wenn die Cornea zu flach oder das Auge zu kurz ist, liegt das im Auge abgebildete Bild hinter der Fläche der Retina. Wiederum erscheint ein beobachtetes Objekt unscharf. Dieser Sehfehler ist als "Hyperopie" bekannt. Schließlich gibt es einen dritten Sehfehler, der mit "Astigmatismus" bezeichnet wird und daher rührt, dass die optischen Flächen im Auge eine gewisse Elastizität aufweisen. Diese Elastizität hat zur Folge, dass es streng genommen mit dem menschlichen Auge überhaupt nicht möglich ist, einen Punkt genau als einen Punkt abzubilden. Vielmehr erscheint das bestmögliche Bild eines Punktes, das im Auge erzeugt werden kann, als Ellipse.

Ophthalmologen und Optiker kennen die aufgeführten Sehfehler. Sie sind in der Lage, diese mit Hilfe von Brillen und Kontaktlinsen korrigieren. Es ist auch möglich, eine "Sehfehlerkorrektur", durch chirurgische Eingriffe am Auge, z.B. durch Einsetzen phakischer Intraokularlinsen in das Auge oder durch sogenannte photo-refraktive Keratomileusis (PRK) vorzunehmen.

Um die Möglichkeiten einer operativen Korrektur von Sehfehlern zu verfeinern, ist es wünschenswert, bei chirurgischen Eingriffen am Auge schnell den Zustand des Auges vor, während und nach einer Operation zu erfassen. Bislang wird nämlich beispielsweise bei Kataraktoperationen dem Patienten eine Intraokularlinse eingesetzt, deren Form aufgrund von präoperativ gewonnenen Patientendaten bestimmt wird. In einer Operation können bislang die optischen Eigenschaften des Auges, an dem operiert wird, nicht überwacht werden. Dies ist problematisch, da sich im Verlauf einer Operation und während einer Wundheilung optische Kenngrößen des Auges, wie Abstände von brechenden Elementen, verändern können.

Wenn es möglich ist, die optischen Eigenschaften des Auges auch während einer Operation zu kontrollieren, kann schnell eine unter Umständen unerwünschte Abweichung von einem vorgeplanten Zustand erkannt und in dem operativen Eingriff angepasst bzw. modifiziert werden. Bei Operationen zur Wiederherstellung der Akkomodation unter Verwendung von injizierbaren Intraokularlinsen ist ein Abgleich der optischen Eigenschaften von Intraokularlinse und Patientenauge sogar notwendig.

Aufgabe der Erfindung ist es, ein Operationsmikroskop bereitzustellen, mit dem bei laufendem Operationsbetrieb, quasi in Echtzeit, der optische Zustand eines gerade operierten Patientenauges zuverlässig und umfassend bestimmt werden kann.

Diese Aufgabe wird durch ein Operationsmikroskop der eingangs genannten Art gelöst, bei dem die Messeinrichtung mit einer Rechnereinheit verbunden ist, welche aufgrund der bestimmten optischen Kenngrößen ein Model für das Patientenauge errechnet und bei dem eine Anzeigevorrichtung zum Anzeigen des errechneten Modells des Patientenauges oder zum Anzeigen von einer oder mehr aus dem errechneten Modell abgeleiteter charakteristischer Größen des Patientenauges vorgesehen ist.

Dabei wird unter einem Modell eines Patientenauges eine Anordnung von optisch wirksamen Flächen und Medien vor einer Bildfläche verstanden, welche die Netzhaut nachbildet, wobei ein dort vorliegender Abbildungsstrahlengang zumindest näherungsweise den natürlichen Gegebenheiten beim menschlichen Auge entspricht. Ein solches Modell eines Patientenauges kann beispielsweise auf dem "Durchschnittsauge von Gullstrad" basieren, das etwa im ABC der Optik, Verlag Werner Dausien, Hanau/Main 1961 auf den Seiten 83 - 85 beschrieben ist. Hier sind für ein "exaktes Augenmodell" und ein "vereinfachtes Augenmodell" die optisch wirksamen Flächen, deren Krümmung und Abstand sowie die Brechzahl von "optischen Elementen" angegeben, für die ein Abbildungsstrahlengang in guter Näherung den natürlichen Gegebenheiten bei einem durchschnittlichen, gesunden Mensch mit emmetropen Auge entspricht. Indem bei dem "exakten Augenmodell" nach Gullstrad oder dem "vereinfachten Augenmodell" Abstände oder Krümmungen nicht als vorgegeben, sondern als auf ein bestimmtes Pätientenauge anpassbare freie Parameter aufgefasst werden, ist es möglich, ein reales Patientenauge mit einem solchen Modell zu beschreiben.

Bei aus dem errechneten Modell abgeleiteten charakteristische Größen kann es sich vor diesem Hintergrund etwa um die Abmessungen des Glaskörpers bei dem entsprechenden Modell, der Augenlänge, den Durchmesser der Augenlinse bei dem Modell, aber auch um die Gesamtbrechkraft des Patientenauges handeln.

Auf dem Gebiet des Optikdesigns ist es bekannt, bei vorgegebenen Parametern bestimmter optischer Elemente einer Anordnung und vorgegebenen Anforderungen, etwa die Lage einer Bildebene, "freie", d.h. nicht vornherein festgelegte Parameter der Elemente mittels Computerprogrammen zu berechnen. Auf dieser Grundlage wird beispielsweise im "American Journal of Ophthalmology, Bnd. 116, S. 63 - 66 (1993)" von Jack T. Holiday vorgeschlagen, die Brechkraft von Intraokularlinsen für ein phakisches Patientenauge zu bestimmen.

Ein besonders gutes Modell für ein Patientenauge lässt sich berechnen, wenn Grundlage der Berechnung zwei oder mehr Kenngrößen des Patientenauges sind. Vorteilhafterweise handelt es sich bei diesen gemessenen optischen Kenngrößen des Patientenauges um solche Kenngrößen, die sich während einer Operation und im darauf folgenden Heilungsprozess möglichst stark ändern. Indem die Messeinrichtung derart aufgebaut ist, dass sie Daten für eine vollständige Charakterisierung der optischen Flächen des Auges, insbesondere das Erfassen von asphärischen Flächenformen und Dezentrierungen ermöglicht, wird die Grundlage geschaffen, mit einem operativen Eingriff am Patientenauge die Sehkraft über die Normalsehkraft hinaus zu verbessern.

In Weiterbildung der Erfindung umfasst die Messeinrichtung ein Kohärenzinterferometer, etwa ein OCT-System. Die Messeinrichtung kann jedoch auch als Wellenfrontsensor ausgebildet sein. Ebenfalls kommt es in Frage, die Messeinrichtung als Refraktometer, vorzugsweise als Refraktometer, als Skiaskop oder als Keratometer auszubilden. Auch eine Messeinrichtung für Laufzeitmessung oder eine Messeinrichtung mit Mitteln zur Durchführung eines Lichtschnittverfahrens ist als Messeinrichtung in dem Operationsmikroskop geeignet. Es versteht sich, dass auch mehrere Messeinrichtungen bei dem Operationsmikroskop vorgesehen sein können. Etwa kann bei dem Operationsmikroskop eine Wellenfront-Messeinrichtung mit einem Skiaskop und eine Refraktometer kombiniert werden. Indem bei dem Operationsmikroskop zusätzlich Mittel zur Messung von nichtoptischen Kenngrößen eines Patientenauges, beispielsweise Mittel zur Messung des Augeninnendrucks vorgesehen sind, ist das Berechnen von besonders zuverlässigen Augenmodellen möglich.

Eine vorteilhafte Ausführungsform der Erfindung ist anhand der Figuren nachfolgend beschrieben.

Es zeigen:
- Fig. 1: ein Operationsmikroskop für die Ophthalmologie zum Bestimmen von optischen Kenngrößen eines Patientenauges; und
- Fig. 2: ein Modell für ein Patientenauge.

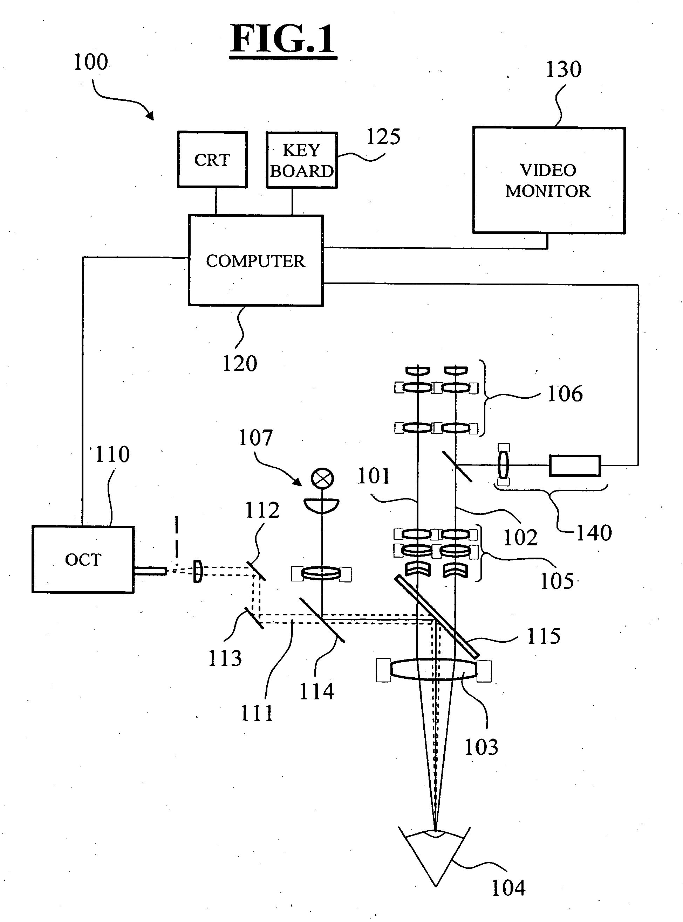

Das Ophthalmo-Operationsmikroskop 100 aus Fig. 1 hat einen stereoskopischen Beobachtungsstrahlengang 101, 102, welcher die Untersuchung eines Patientenauges 104 durch ein Mikroskop-Hauptobjektiv 103 hindurch ermöglicht. Das Ophthalmo-Operationsmikroskop 100 hat weiter ein Zoomsystem 105 und einen Okulareinblick 106. Es umfasst eine Beleuchtungseinrichtung 107, welche durch das Mikroskop-Hauptobjektiv 103 hindurch Beleuchtungslicht für das Patientenauge 104 bereitstellt.

Als Messeinrichtung zum Bestimmen von optischen Kenngrößen des Patientenauges 104 enthält das Ophthalmo-Operationsmikroskop 100 ein OCT-System 110 als Kohärenzinterferometer. Das OCT-System 110 stellt einen Abtastlichtstrahl 111 aus kurzkohärentem Licht bereit, der über verstellbare Scan-Spiegel 112, 113 und Stahlteiler 114 und 115 durch das Mikroskop-Hauptobjektiv 103 hindurch zu dem Patientenauge 104 geführt ist. Das am Patientenauge gestreute Licht des Abtastlichtstrahls 111 gelangt wenigstens teilweise mit dem gleichen Lichtweg zum OCT-System 110 zurück. In dem OCT-System 110 wird dann der Laufweg des Abtastlichts mit einer Referenzstrecke verglichen. Damit kann die genaue Lage von Streuzentren im Patientenauge, insbesondere die Position von optisch wirksamen Flächen mit einer Genauigkeit erfasst werden, welche der Kohärenzlänge des kurzkohärenten Lichts im Abtastlichtstrahl 1.11 entspricht.

Dem OCT-System 110 ist eine Rechnereinheit 120 zugeordnet. Aus den vom OCT-System 110 bereitgestellten optischen Kenngrößen des Patientenauges 104 errechnet die Rechnereinheit 120 ein Modell für das Patientenauge 104. Der Computer 120 ist mit einem Monitor 130 und einer Dateneinspiegelungseinrichtung 140 für das Operationsmikroskop 100 verbunden. Über eine Tastatur 125 des Computer 120 kann die Form der Anzeige des errechneten Models auf dem Monitor 130 oder mittels der Dateneinspiegelungseinrichtung 140 gewählt werden. Auch ist es möglich, die Darstellung des Modells in Form einer Grafik zu wählen oder als Zahlen- oder Zeichensatz, oder selektiv ein, zwei, drei, vier oder mehr aus dem Modell abgeleitete charakteristische Größen auf dem Monitor 130 zur Anzeige zu bringen.

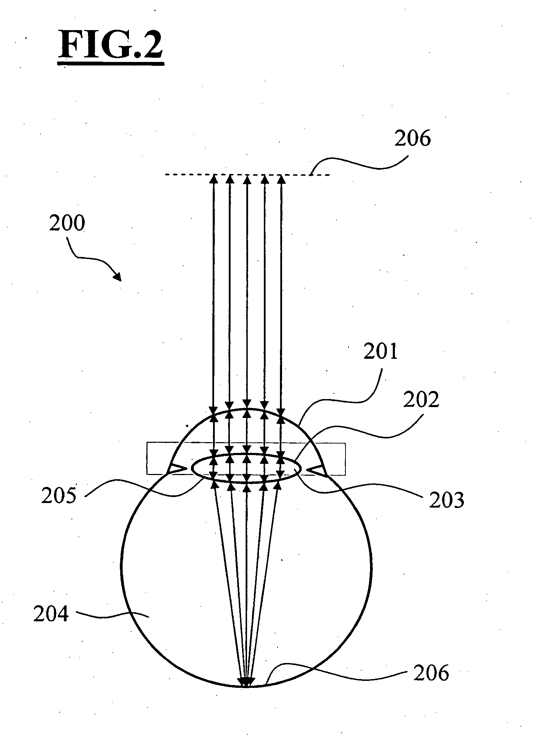

Anhand von Fig. 2 ist ein Modell 200 für ein Patientenauge erläutert. Dieses Modell 200 beruht auf der vereinfachenden Annahme, dass es in einem Patientenauge nur drei optisch wirksame Flächen gibt, eine Außenoberfläche 201 der Kornea, eine Augenlinse 203 mit einer Fläche 202, die zur Kornea 201 weist und die eine Fläche 205 hat, welche zum Glaskörper 204 des Auges orientiert ist.

Mittels des OCT-Systems 110 aus Fig. 1 ist es möglich, bezüglich einer Referenzebene 206 für diskrete Stellen dieser Flächen den exakten Verlauf zu bestimmen. Hiermit kann beispielsweise die optische Größe der Position und die optische Größe des genauen Verlaufs der Außenoberfläche 201 der Kornea und der zur Kornea 201 weisenden Fläche 202 der Augenlinse vermessen und ermittelt werden.

Das Modell geht von der Annahme eines auf unendlich adaptierten Auges aus. Dies bedeutet, dass bei einem rechtsichtigem Auge parallel auf das Auge einfallende Lichtstrahlen am Augenhintergrund 206 fokussiert werden. Aus dieser Zwangsbedingung ergibt sich bei vorgegebenem Verlauf der Außenoberfläche 201 der Kornea und der zur Kornea weisenden Fläche 202 der Augenlinse 203 der Lauf, den die zum Glaskörper 204 weisende Seite 205 der Augenlinse haben muss.

Diese Information wird einem Operateur mit dem in Fig. 1 gezeigten Ophthalmo-Operationsmikroskop bei laufendem Operationsbetrieb zugänglich gemacht. Er ist damit in der Lage, eine in das Auge eingesetzte Intraokularlinse entsprechend anzupassen.

Anstelle oder zusätzlich zum OCT-System kann in das Operationsmikroskop ein Refraktometer, Skiaskop oder Keratometer integriert werden. Ebenfalls kann ein Wellenfrontsensor oder eine Einrichtung zur Laufzeitmessung vorgesehen sein. Mit einem entsprechenden Wellenfrontsensor und einem geeigneten Beleuchtungsstrahl kann bspw. die Brechkraft des Auges ortsaufgelöst bestimmt, d.h. verteilt über die Pupille des Auges an mehreren Orten gemessen werden. Vorzugsweise kann eine solche ortsaufgelöste Brechkraftmessung über den Beobachtungsstrahlengang und die Beleuchtungsteilstrahlengänge im Operationsmikroskop erfolgen. Für eine Messung der Abstände von optisch wirksamen Flächen im Auge können auch diese Strahlengänge, d.h. insbesondere die Strahlengänge, über welche die Brechkraftmessung erfolgt, herangezogen werden. Ferner ist es möglich bei dem Operationsmikroskop auch Einrichtungen zur Messung von nichtoptischen Kenngrößen des Auges vorzusehen, wie etwa eine Messvorrichtung zum Bestimmen des Augeninnendrucks.

## Patentansprüche

1. Operationsmikroskop (100) für ophthalmologische Operationen mit einer Messeinrichtung (110) zum Bestimmen von wenigstens einer optischen Kenngröße eines Patientenauges,
**dadurch gekennzeichnet, dass**
die Messeinrichtung (110) mit einer Rechnereinheit (120) verbunden ist, welche aufgrund der bestimmten wenigstens einen optischen Kenngröße ein Modell (200) für das Patientenauge (104) errechnet, wobei das Modell eine Anordnung von optisch wirksamen Flächen und Medien vor einer Bildfläche ist, welche die Netzhaut nachbildet; und
eine Anzeigevorrichtung (130, 140) zum Anzeigen des errechneten Modells (200) des Patientenauges (104) oder zum Anzeigen von einer oder mehreren aus dem errechneten Modell (200) abgeleiteten charakteristischen Größen des Patientenauges (104) in Form von Augenlänge des Patientenauges und/oder Durchmesser der Augenlinse des Patientenauges und/oder Position der Cornea und/oder Verlauf der Außenoberfläche der Cornea und/oder Verlauf der zur Augenlinse weisenden Oberfläche der Cornea und/oder Verlauf der zur Cornea weisenden Fläche der Augenlinse und/oder Verlauf der zum Glaskörper weisenden Fläche der Augenlinse vorgesehen ist.

2. Operationsmikroskop nach Anspruch 1, **dadurch gekennzeichnet, dass** die Messeinrichtung ein Kohärenzinterferometer (110) umfasst.

3. Operationsmikroskop nach Anspruch 1 oder Anspruch 2, **dadurch gekennzeichnet, dass** die Messeinrichtung einen Wellenfrontsensor umfasst.

4. Operationsmikroskop nach einem der Ansprüche 1 bis 3, **dadurch gekennzeichnet, dass** die Messeinrichtung einen Refraktometer umfasst.

5. Operationsmikroskop nach einem der Ansprüche 1 bis 4, **dadurch gekennzeichnet, dass** die Messeinrichtung einen Skiaskop umfasst.

6. Operationsmikroskop nach einem der Ansprüche 1 bis 5, **dadurch gekennzeichnet, dass** die Messeinrichtung einen Keratometer umfasst.

7. Operationsmikroskop nach einem der Ansprüche 1 bis 6, **dadurch gekennzeichnet, dass** die Messeinrichtung Mittel für Laufzeitmessung umfasst.

8. Operationsmikroskop nach einem der Ansprüche 1 bis 7, **dadurch gekennzeichnet, dass** die Messeinrichtung Mittel zur Durchführung eines Lichtschnittverfahrens umfasst.

9. Operationsmikroskop nach einem der Ansprüche 1 bis 8, **dadurch gekennzeichnet, dass** Mittel zur Messung von nichtoptischen Kenngrößen eines Patientenauges, insbesondere Mittel zur Messung des Augeninnendrucks vorgesehen sind.

10. Verfahren zum Betrieb eines Operationsmikroskops nach einem der Ansprüche 1 bis 9, bei dem
wenigstens eine optische Kenngröße eines Patientenauges (104) gemessen wird;
die wenigstens eine gemessene optische Kenngröße des Patientenauges (104) der Rechnereinheit (120) zugeführt wird, welche auf Grundlage dieser Kenngröße ein Modell (200) für das Patientenauge (104) errechnet, wobei das Modell eine Anordnung von optisch wirksamen Flächen und Medien vor einer Bildfläche ist, welche die Netzhaut nachbildet; und
mittels einer Anzeigevorrichtung (130, 140) das errechnete Modell (200) des Patientenauges (104) als graphische Darstellung oder Zahlenwert angezeigt wird oder mittels der Anzeigevorrichtung (130, 140) aus dem errechneten Modell (200) des Patientenauges (104) abgeleitete charakteristische Größen, in Form von Augenlänge des Patientenauges und/oder Durchmesser der Augenlinse des Patientenauges und/oder Position der Cornea und/oder Verlauf der Außenoberfläche der Cornea und/oder Verlauf der zur Augenlinse weisenden Oberfläche der Cornea und/oder Verlauf der zur Cornea weisenden Fläche der Augenlinse und/oder Verlauf der zum Glaskörper weisenden Fläche der Augenlinse als graphische Darstellung oder als Zahlenwert angezeigt werden.

## Claims

1. Surgical microscope (100) for ophthalmic surgery, comprising a measurement apparatus (110) for determining at least one optical characteristic variable of a patient eye,
**characterized in that**
the measurement apparatus (110) is connected to a computer unit (120) which calculates a model (200) for the patient eye (104) on the basis of the determined at least one optical characteristic variable, the model being an arrangement of optically effective surfaces and media in front of an image surface, which models the retina; and
provision is made for a display device (130, 140) for displaying the calculated model (200) of the patient eye (104) or for displaying one or more characteristic variables of the patient eye (104), derived from the calculated model (200), in the form of eye length of the patient eye and/or diameter of the eye lens of the patient eye and/or position of the cornea and/or profile of the external surface of the cornea and/or profile of the surface of the cornea facing the eye lens and/or profile of the surface of the eye lens facing the cornea and/or profile of the surface of the eye lens facing the vitreous humour.

2. Surgical microscope according to Claim 1, **characterized in that** the measurement apparatus comprises a coherence interferometer (110).

3. Surgical microscope according to Claim 1 or Claim 2, **characterized in that** the measurement apparatus comprises a wavefront sensor.

4. Surgical microscope according to one of Claims 1 to 3, **characterized in that** the measurement apparatus comprises a refractometer.

5. Surgical microscope according to one of Claims 1 to 4, **characterized in that** the measurement apparatus comprises a retinoscope.

6. Surgical microscope according to one of Claims 1 to 5, **characterized in that** the measurement apparatus comprises a keratometer.

7. Surgical microscope according to one of Claims 1 to 6, **characterized in that** the measurement apparatus comprises means for a time-of-flight measurement.

8. Surgical microscope according to one of Claims 1 to 7, **characterized in that** the measurement apparatus comprises means for carrying out a light-section method.

9. Surgical microscope according to one of Claims 1 to 8, **characterized in that** provision is made for means for measuring non-optical characteristic variables of a patient eye, in particular means for measuring the intraocular pressure.

10. Method for operating a surgical microscope according to one of Claims 1 to 9, wherein
at least one optical characteristic variable of a patient eye (104) is measured;
the at least one measured optical characteristic variable of the patient eye (104) is supplied to the computer unit (120) which calculates a model (200) for the patient eye (104) on the basis of this characteristic variable, the model being an arrangement of optically effective surfaces and media in front of an image surface, which models the retina; and
a display device (130, 140) is used to display the calculated model (200) of the patient eye (104) as graphic illustration or as numerical value or the display device (130, 140) is used to display one or more characteristic variables, derived from the calculated model (200) of the patient eye (104), in the form of eye length of the patient eye and/or diameter of the eye lens of the patient eye and/or position of the cornea and/or profile of the external surface of the cornea and/or profile of the surface of the cornea facing the eye lens and/or profile of the surface of the eye lens facing the cornea and/or profile of the surface of the eye lens facing the vitreous humour, as graphic illustration or as numerical value.

## Revendications

1. Microscope d'opération (100) pour opérations ophtalmologiques,
présentant un dispositif de mesure (110) qui détermine au moins une grandeur optique caractéristique de l'oeil d'un patient,
**caractérisé en ce que**
le dispositif de mesure (110) est raccordé à une unité de calcul (120) qui calcule un modèle (200) de l'oeil (104) du patient sur la base de la ou des grandeurs caractéristiques optiques déterminées, le modèle étant un agencement de surfaces et de fluides optiquement actifs situé en avant d'une surface de formation d'image qui imite la rétine,
un dispositif d'affichage (130, 140) qui affiche le modèle (200) calculé de l'oeil (104) du patient ou qui affiche une ou plusieurs des grandeurs caractéristiques de l'oeil du patient (104) déduites du modèle (200) calculé, sous la forme de la longueur de l'oeil du patient, du diamètre de la lentille de l'oeil du patient, de la position de la cornée, de l'évolution de la surface extérieure de la cornée, de l'évolution de la surface de la cornée tournée vers la lentille de l'oeil, de l'évolution de la surface de la lentille de l'oeil tournée vers la cornée et/ou de l'évolution de la surface de la lentille de l'oeil tournée vers le corps vitreux.

2. Microscope d'opération selon la revendication 1, **caractérisé en ce que** le dispositif de mesure comporte un interféromètre à cohérence (110).

3. Microscope d'opération selon la revendication 1 ou la revendication 2, **caractérisé en ce que** le dispositif de mesure comporte un capteur de front d'onde.

4. Microscope d'opération selon l'une des revendications 1 à 3, **caractérisé en ce que** le dispositif de mesure comporte un réfractomètre.

5. Microscope d'opération selon l'une des revendications 1 à 4, **caractérisé en ce que** le dispositif de mesure comporte un skiascope.

6. Microscope d'opération selon l'une des revendications 1 à 5, **caractérisé en ce que** le dispositif de mesure comporte un kératomètre.

7. Microscope d'opération selon l'une des revendications 1 à 6, **caractérisé en ce que** le dispositif de mesure comporte des moyens de mesure de temps de parcours.

8. Microscope d'opération selon l'une des revendications 1 à 7, **caractérisé en ce que** le dispositif de mesure comporte des moyens permettant d'exécuter une opération de découpe par la lumière.

9. Microscope d'opération selon l'une des revendications 1 à 8, **caractérisé en ce qu'**il présente des moyens de mesure de grandeurs caractéristiques non optiques de l'oeil du patient, notamment des moyens de mesure de la pression interne de l'oeil.

10. Procédé de conduite d'un microscope d'opérations selon l'une des revendications 1 à 9, dans lequel
au moins une grandeur caractéristique optique de l'oeil (104) d'un patient est mesurée,
la ou les grandeurs caractéristiques optiques de l'oeil (104) du patient sont amenées à l'unité de calcul (120) qui calcule un modèle (200) de l'oeil (104) du patient sur la base de ces grandeurs caractéristiques, le modèle étant un agencement de surfaces et de fluides optiquement actifs situé en avant d'une surface de formation d'image qui imite la rétine,
au moyen d'un dispositif d'affichage (130, 140), le modèle (200) calculé de l'oeil (104) du patient est affiché sous la forme d'une représentation graphique ou d'une valeur chiffrée ou
au moyen du dispositif d'affichage (130, 140), des grandeurs caractéristiques déduites du modèle calculé (200) de l'oeil (104) du patient sont affichées sous la forme de la longueur de l'oeil du patient, du diamètre de la lentille de l'oeil du patient, de la position de la cornée, de l'évolution de la surface extérieure de la cornée, de l'évolution de la surface de la cornée tournée vers la lentille de l'oeil, de l'évolution de la surface de la lentille de l'oeil tournée vers la cornée et/ou de l'évolution de la surface de la lentille de l'oeil tournée vers le corps vitreux sont affichées sous la forme d'une représentation graphique ou d'une valeur chiffrée.
